# EUROPEAN PATENT APPLICATION

(11) **EP 4 678 148 A1**
(43) Date of publication of application: **14.01.2026**
(21) Application number: 24187205.0
(22) Date of filing: 08.07.2024
(51) Int. Cl.: A61F 13/00, A61F 13/02, A61F 13/0246

(54) **IMPROVED WOUND DRESSINGS AND PROCESS FOR THE MANUFACTURE OF WOUND DRESSINGS COMPRISING SILICONE GEL**

(71) Applicant: Mölnlycke Health Care AB, 431 21 Mölndal (SE)
(72) Inventor: JOHANNISON, Ulf, 438 91 Landvetter (SE)
(74) Representative: Tostmann, Holger Carl

(57) **Abstract**

The present invention relates to an improved process for the manufacture of wound dressings, in particular so-called "island dressings", i.e., dressings that comprise a laminate of layers of different functionalities including (but not limited to) a backing layer, an absorbent pad, a support layer, and a skin or wound facing adhesive layer comprising a silicone gel.

The process according to the invention avoids any perforation of the silicone gel. The process results in a wound dressing with superior performance properties, including improved lateral homogeneity of the wound-facing silicone adhesive layer.

## Description

### Field of the Invention

The present invention relates to an improved process for the manufacture of wound dressings, in particular so-called "island dressings", i.e., dressings that comprise a laminate of layers of different functionalities including (but not limited to) a backing layer, an absorbent pad, a support layer, and a skin or wound facing adhesive layer comprising a silicone gel.

The process according to the invention avoids any perforation of the silicone gel (or a silicone mixture). The process of the invention results in a wound dressing with superior performance properties, including improved lateral homogeneity of the wound-facing silicone adhesive layer.

### Background of the Invention

Silicone gels as the skin/wound facing layer of a wound dressing are generally known (see e.g., WO2009/031948). Providing passages open to fluid flow ("openings") in the generally occlusive (generally non-fluid permeable) silicone gel and the corresponding support layer is of particular relevance for wound dressings since one key purpose of a wound dressing, in particular a wound dressing comprising an absorbent pad, is to take up wound exudate and to allow for rapid and efficient transportation of the wound exudate away from the wound towards the upper layers of a wound dressing, in particular towards the absorbent layer(s) of the wound dressing.

According to processes of the art, the wound contact layer underneath an absorbent layer is perorated to create openings. In terms of manufacture, introducing perforations into a layer of silicone gel is not entirely straightforward since silicone gels generally are viscous thermoset materials that do not necessarily maintain their shape when perforated.

One method for perforating layers used in wound dressings is by way of ultrasonic perforation, i.e. by contacting the to-be-perforated layer or laminate with perforation elements that are subjected to high frequency mechanical vibrations. One such process is disclosed in EP 1 960 164. In another process known from the art (EP 2 382 069), a laminated material including a layer of silicone gel on a melt-blown polyurethane carrier is ultrasonically perforated.

A more sophisticated ultrasonic perforation process for laminates comprising silicone gel is described in EP 3 702 119. Therein, the penetration elements of the ultrasonic device do not simply penetrate the silicone gel layer. Rather, a sacrificial deformable layer is provided, which is deformed in said perforation process such that the deformed portions of said sacrificial deformable layer essentially correspond to the shape of the perforations. This (thermoplastic) deformable layer keeps its shape (i.e., the shape of the perforations) even after the perforation process has been finished and the perforation elements have been retracted from the laminate. However, this laminate comprising the perforated support layer and the silicone gel layer together with the deformed sacrificial layer has to be kept in that "frozen" state for a certain storage time, typically at least 12 hours, in order to assure that the silicone gel, which is not a thermoplastic material, but a thermoset material (i.e., a material that cannot be "melted and cooled" into a new configuration, for example into the configuration of stable perforations) does not (partially) flow back into the perforations created by the perforating elements. This process of silicone gel "flowing back" into a position previously held is also known as "occlusion". After a certain storage time, for example after 12 hours or 24 hours, the silicone gel is generally stable enough so that the sacrificial deformable layer can be removed, and the dressing can be further processed.

Processes of the prior art in which the silicone gel is perforated "directly" by way of ultrasonic perforation using perforating elements are typically associated with the drawback that this "direct" perforation creates a ring-shaped "elevated" area around the perforating elements, as a result of the fact that the thermoset silicone gel has been displaced from its "original" position by the perforation elements and aggregates as elevated "donuts" around the perforation elements. Such elevated ring-shaped areas of silicone gel ("donuts") may persist even after the perforating elements (and a deformed sacrificial layer as described above, if applicable) has/have been retracted and the silicone gel has been fully cured.

Areas of increased height, typically ring-shaped, around the perforations are generally undesirable since the contact area of the silicone gel layer with the surface of the skin or the wound should be laterally as homogenous as possible since this increases the contact area between the skin and the wound dressing.

An alternative process for providing a wound dressing with a perforated silicone gel is disclosed in US 7 470 830, wherein a discrete layer of uncured silicone gel is deposited on the carrier surface of the perforation elements of a perforating device such that the perforation elements extend through the layer of silicone gel. The silicone gel so perforated can then be *transferred* onto other layers of a wound dressing, for example directly on the absorbent core on the wound dressing. This method, however, also involves the "direct" perforation of the silicone gel and memory/occlusion effects cannot be ruled out.

In the same family, US 7 411 109 discusses a variety of possibilities for making perforated silicone gel layers and how to apply a silicone gel layer directly onto an absorbent pad. One of the many options disclosed in US 7 411 109 is to directly apply a silicone mixture onto a heated plate with perforation elements. Once the silicone mixture has cured into a gel, the silicone gel layer can be peeled off the plate with the perforation elements and subsequently *transferred* and applied onto an absorbent pad.

Correspondingly, it is an object of the present invention to provide an improved wound dressings with a wound contacting surface comprising a silicone gel, wherein the above-mentioned drawbacks of occlusion and "flowing back" of silicone gel are avoided or mitigated.

It is a particular object of the invention to avoid any perforation of a curing silicone mixture and of a cured or partially cured silicone gel layer, while providing a wound contact layer that has aligned openings for both the support layer and the silicone gel layer.

### Brief Description of the Figures:

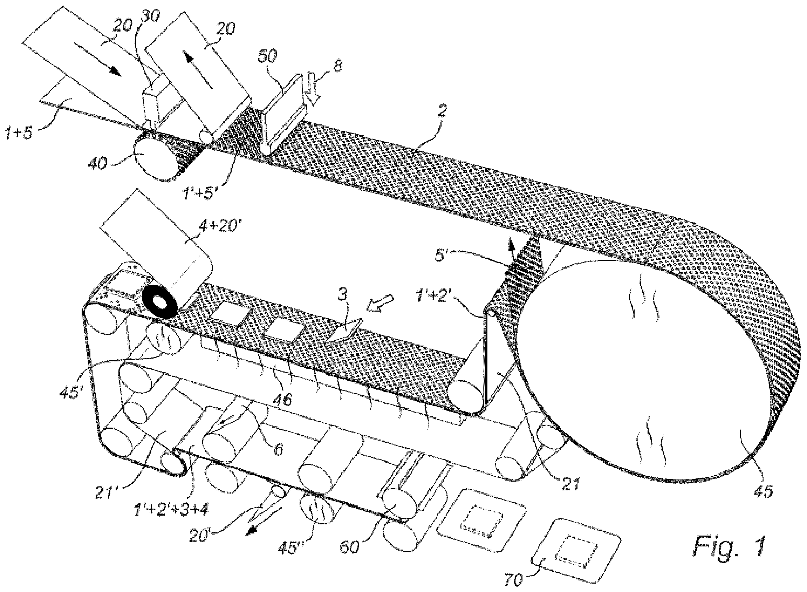

**Figure 1** provides an exemplary overview of aspects of an integrated and continuous process for the manufacture of island dressings, beginning with the feeding of the structural layer (1) and process aid layers and ending with the cutting of the resulting laminate into individual island dressings (70).

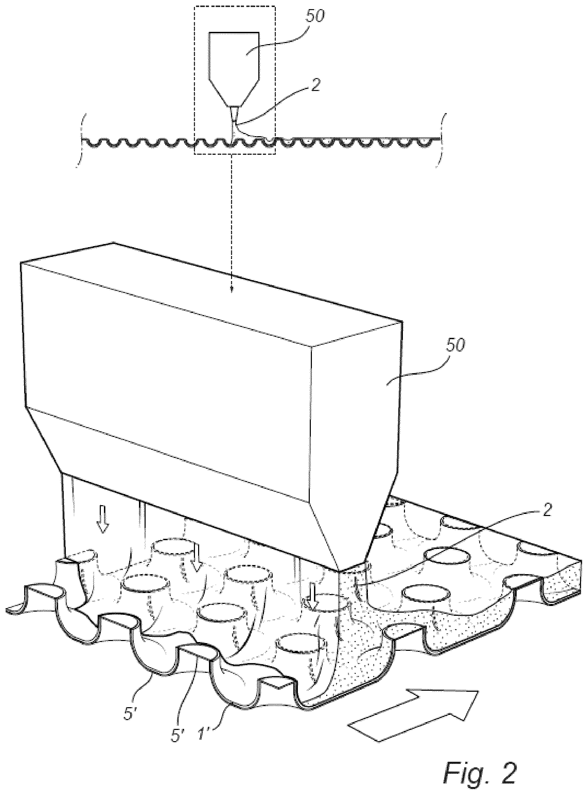

**Figure 2** highlights a further exemplary but more detailed aspect of the overall process, which is of particular relevance to the present invention, namely the step of dispensing an uncured silicone mixture onto a perforated support layer (1'), wherein the perforations in the support layer are "protected" by a deformed template layer (5') that prevents the silicone mixture from entering into or covering the perforations in the support layer (1'). In case "slot" dispensing is performed, as shown in Figure 2, initially, the entirety of the laminate is covered by silicone mixture.

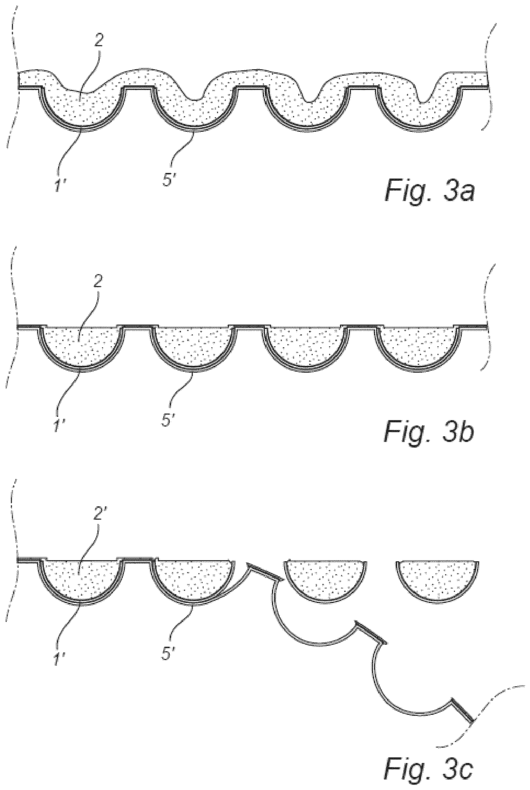

**Figure 3** is also of particular relevance to the present invention and shows a cross-section of an exemplary laminate resulting after the silicone mixture dispensing step (B): The upper panel (Fig. 3a) shows a cross-section of the layers immediately after dispensing: the silicone mixture (2) "evenly" covers the entire deformed template layer (5'). The middle panel (Fig. 3b) shows the same cross-section after the coated laminate (1') + (2) + (5') has traveled along the moving line of the overall process [see Figure 1: relevant is the part of the moving line prior to the curing step on the heated rotating drum (45)]; by then, the silicone mixture (2) has mostly flown into the "valleys" provided between the protruding parts of the deformed template layer (5'); the top parts of the deformed template layers are only covered by a thin film of the silicone mixture. The lower panel (Fig. 3c) shows a cross-section of the product resulting from the delamination step [see also Figure 1: removal of (5') from (1') + (2') immediately after the laminate leaves the heated rotating drum (45)]: the deformed template layer (5') is removed, possibly together with a thin silicone gel layer (now at least partially cured), and the remaining laminate comprises at least partially cured silicone gel (2') with opening essentially aligning with the perforations of the support layer (1').

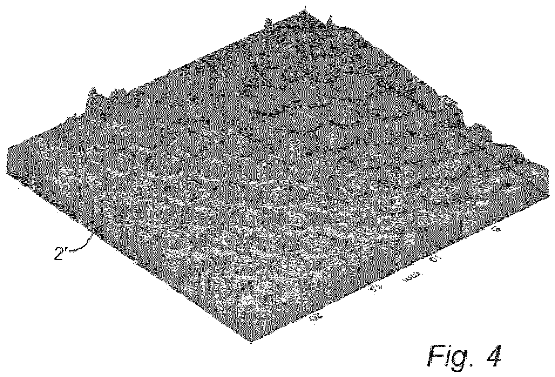

**Figure 4** which is also of particular relevance to the present invention shows a surface scan of a cured silicone gel, wherein the segment from 12 mm to 30 mm shows a cured silicone gel layer that has been perforated by perforating elements (sample made according to the prior art) while the segment from 0 mm to 12 mm shows the openings obtained in accordance with steps (A) and (B) of the present invention (sample with no "donut"-shaped elevations around the opening). For sake of comparison surface scans of the two different samples are shown immediately next to each other.

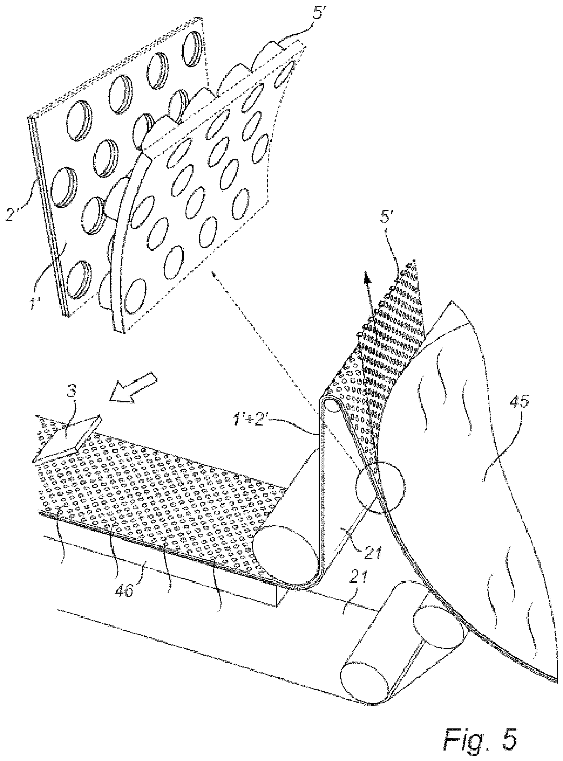

**Figure 5** highlights a further exemplary but more detailed aspect of the overall process, namely the transferring step (D): while the deformed template layer that is no longer needed after the curing step (C) is removed from the continuous process, the cured silicone gel layer on the support layer (both having aligned perforations/openings) is transferred onto a moving (Teflon) belt. This Figure shows a perspective corresponding to the cross-sectional view in Figure 3c.

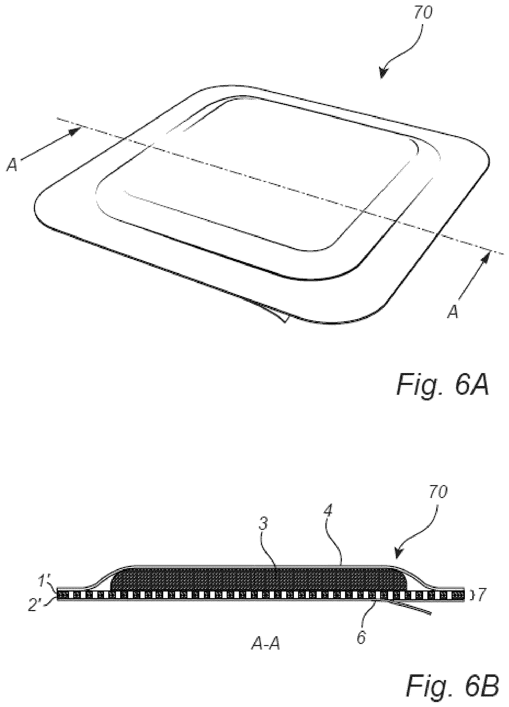

**Figure 6** shows an exemplary embodiment of an island dressing (70) that may be produced using the disclosed process, with the following sequence of layers, starting from the side of the dressing intended to be applied onto the wound: release liner (6) (to be removed prior to use) / silicone gel layer (2') with openings aligning with the perforations in the support layer / perforated support layer (1') / absorbent pad (3) / backing layer (4).

### Summary of the Invention

The above-mentioned objects, and others, are solved by the following process for the manufacture of wound dressings, in particular island dressings, wherein said process at least comprises a feeding and perforation step [in the following also *"perforation step",* (A)], followed by a step (B) of dispensing a mixture of silicone onto the laminate resulting from the perforation step (in the following also *"dispensing step"*), followed by a step (C) of at least partially curing the silicone mixture:
(A) **[feeding and perforation step]** feeding at least a template layer and a support layer through an ultrasonic perforation device,
   which ultrasonic perforation device comprises a plurality of perforation elements, preferably an array of perforation elements, and which device is configured to introduce perforations, in particular an array of perforations, into at least said support layer;
   wherein the perforating elements perforate the support layer, while, at the same time, deform the template layer without perforating the same;
(B) **[silicone mixture dispensing step]** after completion of step (A), dispensing a silicone mixture onto the support layer so that the silicone mixture at least covers the non-perforated parts of the perforated support layer as present on top of the deformed template layer;
(C) **[curing step]** at least partially curing the silicone mixture from step (B) to result in an at least partially cured silicone gel.

In accordance with the present invention, any layer is suitable as a "support" layer as long as the layer is capable to support the silicone mixture and then the cured silicone gel throughout the entire process of manufacture (see Figure 1) and throughout the use of the dressing.

In accordance with the present invention, a silicone "mixture" comprises at least two components that have been mixed prior to dispensing, or are mixed in the dispensing step, wherein the mixture has not been cured at all or only just starts the curing process once mixed.

By contrast in accordance with the present invention, a silicone "gel" has already at least partially cured, i.e. if a fully cured silicone gel has 100% cured, a partially cured silicone gel is cured at least 30%, preferably at least 50%, further preferably at least 60%.

The specific degree of curing is of no relevance to the present invention as long as the silicone mixture as dispensed in step (B) has not already partially cured when dispensed, while the silicone gel after step (C) should be cured to a degree that the skilled person understands will be suitable for the intended use.

Curing times and temperatures that are suitable to reach a desired degree of curing, commensurable with the intended use, are different from silicone mixture to silicone mixture but generally known to the skilled person. The degree of cuing can be determined, for example, by rheometric measurements or by near IR measurements.

The support layer suitably has a melting or softening temperature or range that allows the layer to stay intact and functional during any process step involving energy (heat) intake.

Furthermore, the support layer should be of sufficient flexibility and thickness, among others, so that the resulting wound dressing is suitable for the intended use. The support layer is not only important during manufacture of the dressing but is also an integral part of the dressing at the point of use.

In embodiments, the support layer comprises a polyurethane polymer or is a layer of a polyurethane polymer.

In embodiments the support layer as fed to the ultrasonic perforation device comprises a layer of a polyurethane polymer together with a layer of a polyethylene polymer.

The polyethylene polymer layer supports the comparatively thin polyurethane layer and functions as a processing aid, i.e. is removed in the process and does not become a part of the dressing.

In accordance with the present invention, any layer is suitable as a "template" layer as long as the layer can be *deformed* by perforation elements in an ultrasonic perforation process/device.

The template layer should preferably be adapted to maintain its physical shape after deformation and in subsequent process step(s). Once the template layer is delaminated from the laminate, the template layer plays no further role in the dressing.

In embodiments, the template layer comprises a thermoplastic polymer that has a higher melting point, by at least 10 K, preferably by at least 20 K, than the polymer(s) making up the support layer.

In embodiments, the template layer comprises polypropylene (PP) polymer or is a layer of a polypropylene (PP) polymer.

In embodiments, the overall process is continuous, in particular the template layer and the support layer are fed continuously in step (A).

In the meaning of the present disclosure, "continuous" (feeding) refers to the realization of a process wherein a laminate is continuously moved through the production line/ process steps, and wherein the laminate is successively built up to ultimately result in a continuously moving laminate comprising all layers required for the final dressing. The final individual dressings may then be obtained, for example, by cutting individual dressings out of a continuously moving laminate (see the rotating die cutter device in Figure 1).

In embodiments, the overall process is operated continuously and is preferably operated so that at least 100 dressings, preferably at least 1000 individual dressings result from the overall process, without stopping or having stopped the continuous feeding of step (A).

In the meaning of the present disclosure, a "laminate" refers to two or more sheets or layers (these terms are used interchangeably and synonymously) that are bonded together, for example by feeding one layer over another layer under pressure and/or shear, and/or by heat bonding two or more layers together.

Dispensing an (uncured) silicone mixture onto another layer and subsequently curing the same also results in a "laminate" in the meaning of the present disclosure.

Bonding absorbent pads onto another layer also results in a "laminate" in the meaning of the present disclosure.

In aspects, the laminate does not comprise any "constructive" adhesive between any of the functionally different layers of the laminate, i.e. any adhesive that has the function to adhere one layer onto another layer,
In aspects, the laminate does not comprise any "constructive" adhesive between the backing layer and the support layer.

As mentioned above, the layer of silicone gel present in the wound contact layer does not have the function to adhere one layer onto another layer but rather has the function to adhere the overall dressing to a patient, during use.

As is customary for wound dressings, the wound contacting outer adhesive layer (here: the silicone gel layer) may be protected by a release liner, which is then part of the "laminate" (irrespective of the fact that the release liner is removed prior to use while the other layers of the laminate remain adhered to each other during use and also upon removal of the laminate after use). The presence of a release liner that is adhered to the silicone gel prior to use of the dressing also does not render the silicone gel a "constructive adhesive" as used as a term throughout this disclosure.

In the meaning of the present disclosure, "deforming" a layer, in particular deforming a template layer refers to the process step of changing the shape of the layer in a way that the changed or "deformed" shape persists, even if the elements that have caused the deformation (here: the perforation elements) have been removed from the deformed layer, i.e., are no longer in contact with the layer. Such a lasting or persisting deformation is suitably achieved with a layer made of or comprising a thermoplastic material, in particular in case energy (heat) is applied, as is the case here (ultrasonic perforation or deformation), resulting in a softening or partial melting of the layer.

In the meaning of the present disclosure, "feeding" at least a template layer and a support layer "through" an ultrasonic perforation device, means that these at least two (or more) layers are continuously moved between two opposing elements of an ultrasonic perforation device, in particular between a sonotrode and an array of perforation elements (see more detailed description of an ultrasonic perforation device below), and are brought into contact with each other at least at the point of contact with the perforation elements.

In embodiments (not shown in Figure 1), the perforation elements may be arranged in patterns, for example so that perforations are only present in those parts of the support layer that is in contact with the absorbent pads in the final wound dressing. The functionality of perforations to facilitate uptake of wound exudate for absorption is relevant only (or primarily) for those parts of the wound dressing that actually are in contact ("fluid communication") with an absorbent material. An example of a dressing that has perforations or incisions only or primarily in the area of the wound contact layer that coincides with the absorbent pad can be found, for example, in WO 2016/169948 (see, e.g., Fig. 1) and in WO 2017/081012 (see, e.g. Fig. 3a).

In preferred embodiments of the feeding and perforation step (A), the support layer by itself may be fed as a laminate, for example as a laminate of a layer of a polyurethane polymer (which would be the part of this laminate that ultimately remains in the dressing as a support layer for the silicone gel) and a reinforcing layer of a polyethylene (PE) polymer, which may be removed at a subsequent step of the process.

In further preferred embodiments, a sacrificial paper layer, preferably a paper layer coated with Teflon, is continuously fed together with the support layer and the template layer. The purpose of the paper layer is to facilitate the perforating/deforming by the ultrasonic device by achieving at least one of the following supporting functions: (i) protecting parts of the ultrasonic device, for example the sonicating horn from melting polymer of the support layer; (ii) improving or more evenly distributing heat uptake; (iii) supporting the laminate during mechanical and temperature stress resulting from the exposure to the perforation elements and the ultrasonic energy intake. In Figure 1, such a sacrificial layer is shown as (20) in the upper left part.

In alternative embodiments, instead of a paper film, any suitable (higher melting point) polymer film may also be used as long as at least one of the supporting functionalities (i-iii) disclosed above is achieved.

An exemplary and schematic sketch of a suitable line feeding arrangement is shown in Figure 1, which provides an overview over the entire process, from the feeding and perforation step (A) to the final cutting of the laminate into island dressings. Of particular relevance for the present feeding and perforation step (A) is the upper left part of Figure 1 showing the co-feeding of support layer (1) and template layer (5), wherein the support layer (1) is perforated between the sonotrode (30) of an ultrasonic perforation device and a roll (40) with perforation elements. As a result of this step, support layer (1') is perforated and template layer (5') is deformed but not perforated. A sacrificial processing aid layer (20), for example a Teflon-coated paper layer as discussed above, may be used.

The silicone mixture dispensing step (B) is also illustrated in the upper left corner of Figure (1) [see silicone mixture dispensing unit (50) and the arrow (8) indicating the dispensing of a silicone mixture (2) onto the perforated support layer (1'). This silicone mixture dispensing step (B) is shown in more detail in Figure 2: a silicone mixture dispensing unit (50) dispenses a non-cured (or only minimally curing once dispensed, for example when two components are brought in contact for the first time) silicone mixture (2) onto the perforated support layer (1'), wherein the parts of the deformed template layer (5') that protrude through the perforations of the support layer (1') prevent the silicone mixture from moving into the perforations of the support layer.

In accordance with the present disclosure, the silicone mixture may be dispensed so that it only or primarily covers the "valleys" of the laminate shown in Figures 2 and 3,i.e. only covers the non-perforated part of the support layer as present on top of the deformed template layer, or the silicone mixture may be dispensed as shown in Figure 2, so that (at least temporarily - see discussion of Figure 3 below) also the protruding parts of the template layer are at least partly covered with said silicone mixture.

As an intermediate product of said perforation step (A), a laminate results that comprises a perforated support layer on top of a deformed (but not perforated) template layer, wherein the deformations in the template layer consist of elevated portions, wherein the elevated portions of the deformed template layer protrude through the perforations of the support layer. In essence, the perforations in the support layer are substantially occluded (or "blocked out") by these elevated portions (or protruding parts) of the deformed template layer, thus creating continuous "valley" areas on the surface of the support layer around the elevated portions that protrude the perforations of the support layer (see Figure 2 showing. a schematic depiction of a cross-section of an exemplary laminate resulting from the ultrasonic perforation step).

In embodiments, the silicone mixture in dispensing step (B) is dispensed as an uncured or only partially curing silicone mixture, for example as a two-component silicone mixture, wherein the two silicone components preferably are mixed for the first time in the dispensing unit (prior to this mixing, no curing is possible).

Exemplary and preferred silicone mixtures to be applied in dispensing step (B) are described in more detail below.

In the meaning of the present invention, the silicone mixture is a layer that is on "top" of the support layer in the meaning that during the dispensing step, in the direction of the gravitational force, the support layer is underneath the silicone mixture as dispensed. During use and once the silicone mixture has at least partially cured [i.e., after step (C)], the dressing may be inverted so that the silicone gel may be underneath the support layer in the direction of gravitation, however, the silicone gel is still "on top" of the support layer in the sense that it is coated (and was dispensed) onto the same.

**Figure 3** shows a cross-section of an exemplary laminate resulting after the silicone mixture dispensing step (B): Figure 3a shows a cross-section of the layers immediately after dispensing: the uncured (or only beginning to cure) silicone mixture (2) "evenly" covers the entire support layer (1') and the protruding parts of the deformed template layer (5'). The protruding parts of the deformed template layer (5') occlude the perforations of the support layer (1').

Fig. 3b shows the same cross-section after the coated laminate (1') + (2) + (5') has traveled along the moving line [see Figure 1: relevant is the part of the moving line prior to the curing step on the heated rotating drum (45)]; after moving along the line, for example for 20 seconds, the silicone mixture (2) has mostly flown into the "valleys" provided between the protruding parts of the deformed template layer (5'); the protruding parts of the deformed template layer (5') are only covered by a thin film of the silicone mixture. In this specific embodiment, the silicone mixture "flows" after the dispensing step. In other embodiments, the silicone gel may be dispensed so that it does not significantly flow after dispensing, for example if the silicone gel is directly and only dispensed into the "valleys".

Fig. 3c shows a cross-section of the product resulting from the delamination step, i.e., after curing, for example on a heated rotating drum [see also Figure 1: removal of (5') from (1') + (2') immediately after the laminate leaves the heated rotating drum (45)]: the deformed template layer (5') is removed together with a thin silicone gel layer (now at least partially cured) and the remaining laminate comprises at least partially cured silicone gel (2') with opening essentially aligning with the perforations of the support layer (1').

Dispensing an uncured silicone mixture, onto the non-perforated parts of the support layer (while the perforated parts are not coated since these parts are occluded (or "blocked") by the elevated portions of the deformed template layer), and then at least partially curing this uncured silicone mixture, results in laminate of a support layer having perforations and a coating or layer of a at least partially, preferably fully cured silicone gel that has openings that align with the perforations in the support layer (see Figures 3 and 5).

At the same time, the formation of "elevated" areas ("rings" or "donuts") of a silicone gel around the outer perimeter of the openings/perforations (which typically occurs if the silicone gel is "directly" perforated or otherwise displaced from its original position) is minimized or avoided. As an illustration, see Figure 4 showing a surface scan of a layer of cured silicone gel on a support layer: the left hand side (roughly from 12 mm to 30 mm) shows the surface scan of a sample where perforations on the silicone gel were obtained by prior art ultrasonic perforation, resulting in "donut"-shaped rings around the perforations. A second sample is shown on the right hand side (roughly from 12 mm to 0 mm); here, only the support layer is perforated and the silicone mixture is dispensed as described above (in accordance with the present invention) As is apparent from the juxtaposition of these two different samples, the process according to the present invention allows to obtain a silicone gel surface that has no donut-shaped elevations around the silicone gel. The surface scan was taken with a commercially available FTR surface metrology scanner.

Dispensing (and curing) a silicone mixture onto the non-perforated area of a support layer (while a template layer provides protruding parts that occlude the perforations and create "valley" areas elsewhere) is associated with the advantage that a silicone layer with perforation-like through-holes ("openings") may be created without actually having to perforate or displace the silicone gel, which perforation process is associated with all or some of the potential drawbacks discussed above.

In essence, the silicone gel layer resulting from steps (A) through (C) results in a stable silicone gel layer that is moulded onto the perforated support layer so that the openings in the moulded silicone gel layer align with the perforations of the support layer. The silicone mixture and the resulting silicone gel have not been perforated or otherwise displaced out of their original position achieved in or after step (B).

Since the indentations created in the deformable template layer by way of ultrasonic perforation mimic the shape of the perforating elements, which typically are rod or pin-like, the silicone mixture curing around those indentations will essentially adapt to this shape and thus takes the same shape it would have if it would have been actually perforated by these very same rods or pins.

As an additional advantage of the present invention, compared to processes of the prior art as discussed above, the need for lengthy waiting or "stabilizing" periods is avoided: In prior art process of perforating silicone using a deformable layer and an ultrasonic perforation process, a time interval of at least 24 hours must be observed during which the silicone gel displaced by the protruding perforating elements must be "kept in position" by the (elevated portions of the) deformable "separating" layer to avoid or minimize partial or full return of the silicone gel into its "original" position (prior to being displaced by the perforating elements).

In preferred embodiments, the curing step may be at least partially performed on a rotating drum:
(C) **[curing step]** curing the silicone mixture from step (B) to result in an at least partially cured silicone gel, while the laminate from step (B) at least partially rotates on a heated rotating drum;

Specifically performing at least, a part of the overall curing step on a heated rotating drum is associated with the advantage that improved thermal contact allows for a more efficient curing process. Furthermore, steps (A) through (C) can be suitably integrated into a continuous overall process for making island dressings as described in more detail below.

In embodiments of the invention, the curing time is determined by (and in turn can be controlled by setting) the temperature of the heated rotating drum, the diameter of the heated rotating drum, and the speed of rotation of the heated rotating drum, among others.

An example of this curing process is shown in Figure 1 [see large heated rotating drum (45) on the right side of Figure 1]: A laminate comprising deformed template layer (5') with the perforated support layer (1') and a silicone mixture (2) coated thereon is heated on a rotating drum (45). In the drawing, generally, "flame" symbols (here: three such flame symbols) indicate heating by contact.

In conventional processes for making wound dressings comprising silicone gels that need to be cured, said curing is typically performed on stationary heated plates and is often performed outside a continuously running system. For example, the laminate comprising the to-be-cured silicone is taken off the continuously running system and transferred onto a separate support layer that is pulled over heated plates.

Furthermore, the present process is advantageous in that curing and "perforation" are performed in line and as part of an integrated process. As discussed above, no separate perforation step on an already cured silicone gel is required, but rather the "perforation" happens "automatically" as a result of the dispensing step (B) and the curing step (C).

In preferred embodiments, the laminate comprising the to-be-cured silicone mixture, for example a mixture of a two-component system, is continuously cured in-line while the overall processing of the laminate continues (see also Figure 5). Therefore, not only is the overall process indeed integrated and devoid of interruptions or elements of batch processing, but, in addition, separate or different process lines or pieces of equipment such as heating plates or separate oven units is/are avoided (rotating rolls or drums are part of the overall process anyway).

As a further advantage, as outlined above, the rotating drum can be flexibly configured to adjust a desired curing time (e.g., by way of varying the diameter of the drum, the rotating speed, the temperature of the drum etc.).

The use of a heating drum for curing the silicone gel as is particularly advantageous in a continuous process. Running the laminate with the coated silicone gel on a rotating drum reduces the friction against the heating surface. Close contact to the heated surface can be established by "tensioning" the laminate, for example by the use of sets of rotating rolls pressing against the laminate. The process according to the invention avoids the use of a further sacrificial tissue layer at the curing stage. Such a sacrificial layer or a web is needed in conventional processes in which a polymer laminate coated with silicone gel is moved, on a further support layer, over heated plates.

The curing temperature typically depends on the silicone gel to be cured, for example its composition or the thickness, and on the specific set-up of the rotating drum. Any temperature that suitably cures the silicone gel to a degree so that the silicone gel can be used in a wound dressing is within the scope of the present invention.

Preferred curing temperatures as generally applicable to step (C), for example as the ambient temperature around the silicone mixture to be cured, and as particularly preferably defined by the temperature on the surface of the heated rotating drum that is in contact with the laminate, are from 100°C to 170°C, preferably from 120°C to 150°C.

The curing time typically also depends on the silicone gel to be cured, for example its composition or the thickness, and on the specific set-up of the rotating drum. Any time range that suitably cures the silicone gel to a degree so that the silicone gel can be used in a wound dressing is within the scope of the present invention.

Preferred curing times are in the range of from 20 seconds to 10 minutes, preferably from 20 seconds to 60 seconds.

Once the curing process is completed, in accordance with the specifications of the process, the perforated support layer coated with the at least partially cured silicone gel can be separated from the deformed template layer and, if applicable, from any further reinforcing layer that may have been co-fed with the support layer (and/or template layer) in the feeding and perforating step (A) (see Figure 1 and preferred embodiments discussed above).

In aspects of an integrated process for the continuous manufacture of wound dressings, in particular island dressings, the process further at least may comprise a transferring step:
(D) **[transferring step]** removing the laminate from step (C) from the heated rotating drum while separating the perforated support layer with the at least partially cured silicone gel of step (C) from the deformed template layer and, if present, from other optional layer(s), and transferring said separated laminate comprising the at least partially cured silicone layer on the perforated support layer onto a moving belt;

In aspects, the transferring step is:
(D') **[transferring step]** separating the perforated support layer with the at least partially cured silicone gel of step (C) from the deformed template layer and, if present, from other optional layer(s), and transferring said separated laminate comprising the at least partially cured silicone layer on the perforated support layer onto a moving belt;

In aspects, the moving belt is or comprises a composite material that is compatible with temperatures of up to 150°C, preferably up to 200°C (i.e. has high heat resistance) and/or that has a static coefficient of friction ("COF") of below 0.15, preferably below 0.1 (corresponding to lubricated steel)

In preferred aspects, the moving belt is or comprises a polyfluorinated polymer, preferably polytetrafluorethylene (PTFE or "Teflon"), particularly preferred is or comprises fiberglass reenforced Teflon. Teflon has COF of 0.04.

In preferred aspects, said moving belt rotates continuously in the overall system (see Figures 1 and 5). The transferring step is shown in the central part of Figure 1 and shown in more detail in Figure 3c and in Figure 5: The deformed template layer (5') as tangentially coming off a rotating heated drum (45) is delaminated from the support layer (1') with the now at least partially, preferably fully cured silicone gel layer (2'). The deformed template layer may be discarded and is, at any rate, not part of the final dressing. Other processing aid layers may also be delaminated at this stage. The support layer (1') with the at least partially cured silicone gel layer (2') is concurrently transferred onto a moving belt (21). The following process steps are then performed on the laminate as transported onto the moving belt.

As already alluded to above, concurrently with the transfer of the laminate comprising the at least partially cured silicone layer on top of the perforated support layer onto the moving belt, which transferred laminate is the laminate that will ultimately end up in the final product (wound dressing), any further layer(s) no longer needed, in particular the deformed template layer, but also other optional layers that may have been introduced previously as processing layers, for example a reinforcing layer for the supporting layer, may be removed at that stage.

As a result of this transferring step, the silicone gel is in direct physical contact with the moving belt, preferably a moving belt comprising Teflon, and can be easily removed in a subsequent step further down the line.

The next layer in the sequence of the laminate (on top of the silicone gel layer in contact with the moving belt) is: the perforated support layer that has two interfaces, one interface with the silicone gel layer coated thereon and one "outward" facing interface that is capable of receiving the absorbent pads in the subsequent application step (see below).

In aspects of the overall process, an application step (E) follows the transferring step (D):
(E) **[application step]** continuously applying absorbent pads onto the laminate from step (D), said laminate comprising at least a perforated support layer and a silicone gel layer, wherein the absorbent pads are applied onto the side of the support layer that is not coated with silicone gel, wherein said absorbent pads are at least partially heat bonded to said side of the support layer not coated with silicone gel;

The application step (E) is illustrated in Figure 1, central part: absorbent pads (3) are applied onto the laminate with the perforated support layer facing the direction of application pads (3). The entire laminate comprising at least (1') + (2') and sitting on top of the moving belt (2) is heated from below along the heating zone (46) [again: "flame"-symbols indicate contact heating)]

In aspects, the at least partial heat bonding of the absorbent pads to the support layer is achieved by heating the moving belt from below so that the temperature of the support layer is in the range of from 100°C to 180°C, preferably from 120°C to 160°C.

Without wishing to be bound by theory, it is believed that if the moving belt is heated from underneath, i.e. from the side that is not in direct physical contact with the laminate, to a temperature in the range of from 100°C to 180°C, preferably of 120°C to 160°C, the support layer is sufficiently softened that at least a partial heat lamination, preferably a full heat lamination, occurs between at least parts of the absorbent pads and the support layer, for example by way of "physical" bonding between the two surfaces (lower surface of the backing layer, upper surface of the support layer).

In a subsequent or concurrent laminating step, a backing layer is continuously applied onto the laminate including the absorbent pads from the previous application step (E) and the backing layer is at least partially heat bonded at least to the support layer:
(F) **[laminating backing layer step]** continuously feeding a backing layer onto the laminate from step (E), wherein the backing layer is applied onto the absorbent pads and the side of the support layer that is not coated with silicone gel, wherein the backing layer is at least partially heat bonded to said side of the support layer that is not coated with silicone gel.

This laminating backing layer step is illustrated in Figure 1, lower left part of the Figure: a backing layer (4) [together with a processing aid layer (20')] is continuously applied onto the laminate comprising perforated support layer (1'), silicone gel layer (2'), wherein absorbent pads are arranged onto this laminate. The laminate continuously moves as transported by the moving belt (21). In this exemplary embodiment, laminate, backing layer and belt are all fed together and pressed together between two rolls, wherein the lower roll (45') is heated (see "flame" symbols). The processing aid layer (20') is removed further down the line [after application of the release liner (6); see lower left part in Figure 1]. The purpose of this processing aid layer is to support and stabilize the comparatively flexible backing layer.

In aspects, the at least partial heat bonding of the backing layer to the support layer is achieved by heating the moving belt, or a lower roll, or both, to a temperature in the range of from 100°C to 180°C, preferably of 120°C to 160°C.

Without wishing to be bound by theory, it is believed the support layer is sufficiently softened by this heating that at least a partial heat lamination, preferably a full heat lamination, occurs between at least parts of the backing layer and the support layer by way of "physical" bonding between the two surfaces (lower surface of the backing layer, upper surface of the support layer).

In aspects, the at least partial heat bonding of the backing layer onto the support layer is also/ further facilitated by exerting pressure onto the laminate comprising the backing layer and the support layer, preferably by way of pressing the laminate between an upper roll and a lower roll (for an illustration see discussion of Figure 1 above).

In preferred aspects, exerting pressure onto the laminate also comprising the absorbent pads, preferably by way of pressing the laminate between an upper roll and a lower roll, further facilitates the at least partial heat bonding between the support layer and the absorbent pads.

In aspects, the upper roll is softer than the lower roll.

In aspects, the lower roll underneath the laminate comprising the silicone gel / perforated support layer / absorbent pad is heated to a temperature in the range of from 100°C to 180°C, preferably from 120°C to 160°C

Step (F) of heat bonding the backing layer onto the support layer may be performed at least partly *concurrently* with step (E) of heat bonding the absorbent pads onto the support layer.

In aspects, the temperature of the lower roll underneath the moving belt, and the speed of rotation of the lower roll and/or the pressure exerted by pressing the laminate between said upper and said lower roll is/are adjusted so that the backing layer and the backing layer are at least partially heat bonded (laminated) onto the support layer, optionally so that the backing layer is also at least partially heat bonded onto the absorbent pads.

The two steps (E) and (F) involving heat bonding are illustrated in Figure 1: The absorbent pads (3) are applied onto the perforated support layer (2') while the moving belt is heated from underneath in the heating zone (46). Full or partial heat bonding (= lamination) of the absorbent pads onto the support layer is achieved in that heating zone.

Further heat bonding of the absorbent pads onto the support layer may be achieved in the subsequent step of adding the backing layer (6) wherein the entire laminate, including the absorbent pads, is pressed between two rotating rolls, wherein at least the lower roll (45') is heated. Heat and/or pressure achieve heat bonding/lamination of the backing layer (4) onto the perforated support layer (1'). Optionally and to a varying degree (depending on temperature, pressure etc.), heat bonding/lamination of the backing layer onto the absorbent pad and/or of the absorbent pad onto the support layer may also be achieved in this step.

As a result of these laminating/ heat bonding steps, the resulting laminate as transported on the moving belt has the following sequence of layers beginning with the layer that is in physical contact with the moving belt: silicone gel with openings that essentially correspond to the perforations of the support layer / perforated support layer / absorbent pads / backing layer covering the absorbent pads and the outwardly facing side of the support layer in the border region.

As a result of step (F), the backing layer is at least partly heat bonded to the support layer in the border region around the absorbent pad, i.e. the area that does not comprise an absorbent pad. In the final product (wound dressing), the absorbent pad has a smaller area than the backing layer and the support layer, if viewed from top or form below (see Figure 6).

As is the case for all embodiments of the present disclosure, if a specific sequence of layers is disclosed, any other number or kind of layers may be present at any position, as long as the sequence of the specifically mentioned layers is maintained.

For example, a further layer may be present between support layer and absorbent pad/backing layer. In particular, the absorbent pad may include several sublayers (e.g., the absorbent pad may be a laminate of a foam layer and a fibrous layer).

In aspects, the backing layer comprises or consists of a polyurethane polymer, in particular a film of a melt-blown polyurethane polymer, which is optionally fed together with a further supporting layer in step (F), which further supporting layer preferably comprises or consists of polyethylene.

Without wishing to be bound by theory, the heat provided by the rotating roll underneath the moving belt (and therefore also underneath the laminate) is sufficient to soften the materials in question, in particular the support layer and possibly, at least to some extent, also the backing layer so that it is possible to partially or fully heat-bond the backing layer onto the support layer.

In preferred aspects, the upper roll is made of a comparatively soft material, for example a polymeric material, whereas the lower roll is made of a comparatively solid material, for example a heat-conducting metal.

It is also within the scope of the present disclosure that in step (F), the backing layer is also at least partly heat bonded onto the absorbent pads. Advantageously, the upper roll is also heated in that case.

The fact that a moving belt is used in the overall process is advantageous for any process step that involves heat bonding/laminating, such as step (E) or step (F), since the moving belt facilitates an even transfer of heat from below, for example from a heated roll to the laminate that is to be bonded together.

The fact that the backing layer and the support layer are heat-bonded with each other, as well as that the absorbent pad is at least partly heat bonded onto the support layer is associated with the advantage that no construction adhesive is needed to laminate those two layers together in the border area around the wound pads.

Any introduction of a further layer, in particular a layer of an adhesive may create a hindrance to fluid flow, be it a wound exudate that is to be transported away from the wound, or be it vapor that needs to be transported out of the wound dressing by way of evaporation through a vapor-permeable backing layer.

In aspects, after laminating step (F), a cooling and delamination step (G) is implemented, in which step the laminate resulting from step (F) is cooled down to a temperature range of from 15°C and 40°C, preferably cooled down to a range of from 18°C to 25°C, wherein this cooled laminate is subsequently removed from the moving belt, i.e. delaminated from the moving belt.

After said cooling and delamination step (G), the moving belt, without any laminate deposited thereon, further moves, preferably rotates in the overall continuous process and - further down the line - is brought into contact with the laminate coming from the heated rotating drum in step (D)

This delamination step is also schematically shown in Figure 1 [lower left most corner,just underneath roll (45')]: the moving (Teflon) belt (21') is separated from the laminate comprising all layers that ultimately end up in the dressing: silicone gel layer (2') on perforated support layer (1'), which is heat laminated with the backing layer (4) and the absorbent pad (3).

Using a pretensioned moving belt and delaminating the laminate from said belt allows for an "inline tackiness control", i.e. allows for measuring the force required for delaminating the silicone gel off of the essentially inert surface of the moving belt. This provides a tangible parameter that allows to evaluate the adhesion force ("tackiness") that the silicone gel will also have on the skin. Based on the results of this inline tack control, portions of the continuously produced laminate can be discarded, if their measured tackiness is not in line with predefined specifications.

A further advantage associated with implementing an integrated and continuous process is that the use of a moving belt, in particular use of a Teflon belt that is reinforced by fiberglass, allows to synchronize process steps over a significant length of the continuously moving belt. For example, since the belt is comparatively strong and stiff, the tension of the belt as applied by the rotating rolls may be adjusted to further improve synchronization of steps and process control.

The materials that preferably make up the moving belt have a low coefficient of friction vis-à-vis other materials, in particular vis-à-vis silicone gel. Since it is preferred that a laminate comprising silicone gel is transported in lateral direction in a continuous process by way of a moving belt, it is advantageous that it is easily possible to delaminate the overall laminate from the moving belt to implement the final process steps. If a continuously moving belt of a more conventional plastic material were used, for example a conventional somewhat elastic plastic material, not only would synchronization and process control between the different process step be negatively affected, but also a further processing aid, for example a Teflon coated paper would be needed to ensure suitable delamination.

The movement of the belt through steps (D) to (G), or subsets thereof, may be repeated as often as desired.

After step (G), the laminate is subjected to final processing steps that may be implemented advantageously to obtain the desired wound dressings of suitable for use.

In a further optional step (H), a release liner is added onto the outside facing side of the silicone gel as coated onto the supporting layer, i.e. onto the interface that had - up to this point - been in contact with the moving belt.

The step of adding a release line is shown in the lower left part of Figure 1: after separating the laminate from the moving belt, release liner (6) is added to the silicone gel side of the dressing (that has just come off the moving belt) between two counterrotating rolls. Immediately after said step of adding a release liner (and thus covering and protecting the silicone gel layer), the support layer (20') that was added together with the backing layer (4) further upstream (see discussion above) is removed.

An optional (further) laminating of the backing layer to the absorbent pad may be achieved with heated roll (45").

In aspects, the release liner is continuously applied onto the laminate by way of feeding the same between two counterrotating rolls. At this point, in case any further reinforcing layer may still be present, for example a PE layer that has been fed together with the backing layer, is continuously removed resulting in a laminate of the following sequence of layers: release liner / silicone gel / perforated supporting layer / absorbent pad / backing layer.

In an optional further step (I), assuming that heat bonding the backing layer against the absorbent pad is desirable, the backing layer may also be at least partially heat bonded onto the absorbent pad, in particular between two rotating rolls, wherein the lower roll, i.e. the roll in direct contact with the backing layer, is heated to a temperature in the range of from 100°C to 180°C, preferably from 120°C to 160°C (see discussion of Figure 1 above).

Heat bonding the backing layer against the absorbent pad - either as achieved in the laminating step (F) in which the backing layer is heat bonded onto the support layer and/or as achieved in separate step (I) as shown in Figure 1, or both, - is associated with the advantage that a stable direct contact between the backing layer and the absorbent pad facilitates continuous evaporation of water vapor (e.g. from absorbed wound exudate) through the backing layer, which is preferably a moisture vapor-permeable membrane, also and in particular in the wet state during use. Thus, maintaining direct physical contact between the absorbent pad and the backing layer facilitates for the transport of water vapor from the inside of the dressing to the outside of the dressing during use of the dressing.

By providing a heat bond between the backing layer and the absorbent pad such that the absorbent layer is in close or direct physical contact with the backing layer, the overall moisture vapor transmission rate (MVTR), also known as water vapor transmission rate (WVTR), of the dressing may be increased.

On the other hand, for some uses, for example if wound dressings are to be applied to curved areas of the human body, high flexibility of the overall dressing may be desirable and it may not be desirable that the dressing is too stiff, for example by way of providing a strong bonding between the backing layer and the absorbent pad.

In such a case, it may be advantageous to not heat bond the backing layer (also) to the absorbent pad. In aspects, the backing layer is only partly heat bonded to the absorbent pad, e.g. in a pattern, thus balancing the above discussed functionalities of moisture vapor transmission rate and flexibility.

By using the continuous process as disclosed herein, which allows to continuously adjust the process parameters, such as the pressure between the rolls, temperature of the rolls, inline speed etc., the heat laminating between the backing layer and the absorbent pad can be continuously adjusted and optimized depending on the desired specifications of the final product.

In a further optional step of the overall integrated process, the laminate from the previous step(s) (G) or (H) or (I) is cut into individual wound dressings comprising a release liner, a silicone gel layer with openings that essentially coincide with the perforations of a perforated support layer, said perforated support layer, a absorbent pad, and a backing layer, preferably, the cutting is achieved by means of a rotating die cutter ("RDC" - see (60) in Figure 1). Said rotating die cutter allows to continuously cut individual wound dressings (70) out of a laminate.

A further aspect of the present invention relates to a **wound dressing,** in particular an island wound dressing, said dressing at least comprising:
- at least one absorbent pad;
- a vapor permeable backing layer overlaying said absorbent pad;
- a perforated support layer underneath said absorbent pad, wherein said backing layer and said support layer extend beyond a periphery of said absorbent pad to define a surrounding border portion along said periphery of the absorbent pad;
- a silicone gel layer on the side of the support layer that is opposite to the side that contacts the backing layer, wherein the silicone gel layer has openings that correspond to the perforations of the perforated support layer;
wherein said silicone gel layer has been moulded onto said support layer.

In embodiments the dressing optionally comprises a release liner covering the silicone gel layer, wherein the release liner is suitable to protect the silicone gel layer prior to use as a wound dressing.

In embodiments, the silicone gel layer with openings has been obtained by applying a silicone mixture onto a perforated support layer and subsequently curing said silicone mixture to result in a at least partially cured silicone gel,

In preferred embodiments, the silicone gel layer has not been perforated, in particular has not been exposed to any perforating elements.in particular no mechanical perforation elements and no ultrasonic perforation elements.

An exemplary island dressing (70) is shown in Figure 6: Backing layer (4) overlays absorbent pad (3) and is heat laminated to the perforated support layer (1'). Support layer (1') and silicone gel layer (2') together form the wound contact layer (7). Backing layer (4) and wound contact layer (7) form a border portion or border region around the "island" of the absorbent pad (particularly prominently visible in the upper perspective view). The openings of the silicone gel layer (2') align with the perforations of the support layer (1').

In so-called island dressings, this border region may have a width of 1 cm or 2 cm, around the entire circumference of the central absorbent pad.

In embodiments the support layer is perforated only in those parts of the support layer that are in contact with an absorbent pad.

In preferred embodiments, the wound dressing is further characterized in that the dressing overall does not contain any adhesive other than the silicone gel layer, in particular does not comprise any acrylic adhesive.

In embodiments, the thickness of the silicone gel layer does not vary by more than 10%, preferably not by more than 7%, further preferably not by more than 5% across the entire lateral area of the silicone gel layer.

In embodiments, the openings in the silicone gel layer are circular and have a diameter of from 0.5 mm to 2 mm.

In embodiments, the openings in the silicone gel layer are arranged in an array, wherein the center of each opening is spaced apart from a neighboring center of another opening by a distance of from 2 mm to 4 mm.

In aspects, the backing layer and the support layer are at least partially heat laminated together so that the adhesion between these two layers exceeds the peeling strength required to remove the entire wound dressing from the wound.

As discussed above, directly dispensing a silicone mixture into the "valleys" of the non-perforated parts of a support layer instead of perforating a silicone mixture as applied on a support layer, or instead of moving a silicone mixture or a silicone gel out of its original position by way of exposing the silicone mixture/gel to protruding perforating elements, results in a silicone gel layer that, when cured, is evenly spread on a perforated support layer and has openings aligning with said perforations, without, however, the silicone mixture or the silicone gel itself ever being subjected to a perforation step or a "moving out of its original place"- step.

In essence, the silicone mixture [of step (B)] is "moulded" into its position ultimately desirable for the dressing, by first allowing the silicone mixture to flow and spread out on the support layer and around the (occluded) perforations in the support layer and then at least partially curing, preferably fully curing the silicone mixture. This results in the silicone gel layer with openings that align with the perforations in the support layer.

This conceptually different (from perforation) method of creating a cured silicone gel layer on top of a perforated support layer (wherein said silicone gel layer has openings that essentially coincide with the perforations of the support layer) avoids any "pushing away" of the thermoset silicone gel material (during or post curing) resulting in a "counterforce" that leads to a partial or complete reclosing of openings that have been created by "pushing away" silicone gel, for example by way of protruding perforating elements.

According to processes known from the art, this "memory effect" of silicone gel tending to flow back into its original position, i.e. its position prior to perforation as known , for example from EP 3 702 119, may be circumvented or mitigated by waiting for long periods after the perforation step, for example 12 hours, or 24 hours, or 48 hours with the perforation elements still within the silicone gel layer or by using processing aid layers, such as a sacrificial layer, that has been deformed together with the silicone gel.

By contrast, the present process not only reduces the time needed to achieve a laterally homogenous silicone gel layer with stable openings, but also avoids the formation of ring-shaped elevated areas ("donuts") around the openings at which perforation elements had protruded the silicone gel layer, since the silicone gel is never pushed outside its original position but rather is dispensed onto a support layer comprising openings which are blocked by elevated portions (protruding parts) of the deformed template layer and thus creating suitable receptacles ("valleys") around the circumferential shape of the elevated portions of the deformed template layer.

In further aspects and as mentioned above, avoiding the presence of any "construction" adhesive, between the backing layer and the support layer and/or the absorbent pads and the between the support layer and the absorbent pads, in a wound dressing, is associated with several advantages, one of them being that moisture transmission may be improved, another advantage being that less components and materials need to be used in the process and less materials end up in the product.

More specifically, any adhesive layer that is present between two functionally separated layers of a wound dressing, for example the backing layer and the absorbent pad, creates another interface that must be penetrated for transport, for example for transport of water vapor, through the backing layer away from the wound into the atmosphere. Any hindrance to fluid flow may impair the overall performance of the dressing.

Furthermore, if adhesives are used between layers in the manufacturing process of a wound dressing, this is typically associated with the presence of an undesirably high amount of water that needs to be removed from the dressing prior to final packaging. In processes of the art, removing residual water from acrylic adhesive is achieved in hot air ovens / convection ovens. In the process of the present invention, the use of separate equipment needed for the drying of parts of the wound dressing or the entire wound dressing, for example in energy intensive ovens can be avoided or minimized since no acrylic adhesive is used in the disclosed process.

The present disclosure also relates to the following embodiments which may be combined with each other and/or with embodiments as described above:
1. Process for the manufacture of wound dressings, said process at least comprising the steps of:
   (A) feeding at least a template layer and a support layer through an ultrasonic perforation device,
      which ultrasonic perforation device comprises a plurality of perforation elements, preferably an array of perforation elements, and which device is configured to introduce perforations, in particular an array of perforations, into at least said support layer;
      wherein the perforating elements perforate the support layer, while, at the same time, deform the template layer without perforating the same;
   (B) after completion of step (A), dispensing a silicone mixture onto the support layer so that the silicone mixture at least covers the non-perforated parts of the perforated support layer as present on top of the deformed template layer;
   (C) at least partially curing the silicone mixture from step (B) to result in an at least partially cured silicone gel, preferably while the laminate from step (B) at least partially rotates on a heated rotating drum;
   (D) optionally removing the laminate from step (C) from the heated rotating drum while separating the perforated support layer with the at least partially cured silicone gel of step (C) from the deformed template layer and, if present, from other optional layer(s), and transferring said separated laminate comprising the at least partially cured silicone gel layer on the perforated support layer onto a moving belt;
   (E) optionally continuously applying absorbent pads onto the laminate from step (D), said laminate comprising at least a perforated support layer and a silicone gel layer, wherein the absorbent pads are applied onto the side of the support layer that is not coated with silicone gel, wherein said absorbent pads are at least partially heat bonded to said side of the support layer not coated with silicone gel;
   (F) optionally continuously feeding a backing layer onto the laminate from step (E), wherein the backing layer is applied onto the absorbent pads and the side of the support layer that is not coated with silicone gel, wherein the backing layer is at least partially heat bonded to said side of the support layer that is not coated with silicone gel.
2. The process of embodiment 1, further comprising at least one of the following steps, preferably at least further comprising steps (G) and (H):
   (G) cooling the laminate resulting from step (E) or from step (F) down to a temperature in a range of from 15°C to 40°C, preferably from 18°C to 25°C, wherein the cooled laminate is subsequently removed from the moving belt;
   (H) continuously feeding and adding a release liner onto the silicone gel layer of the laminate as removed from the moving belt in step (G),
   (I) at least partially heat bonding the backing layer onto the absorbent pad, optionally between two rotating rolls, wherein the upper roll, i.e. the roll in direct contact with the backing layer, is heated to a temperature in the range of from 120°C to 160°C.
3. The process according to any of the preceding embodiments, wherein the support layer is fed as a laminate, optionally as a laminate of polyurethane (PU) together with a reinforcing layer of polyethylene (PE).
4. The process according to any of the preceding embodiments, wherein the silicone mixture in dispensing step (B) is dispensed as an uncured two-component silicone mixture.
5. The process according to any of the preceding embodiments, wherein the curing time in step (C) is determined by adjusting one of the following, optionally by adjusting all of the following: temperature of a heated rotating drum, diameter of a heated rotating drum, speed of rotation of a rotating drum.
6. The process according to any of the preceding embodiments, wherein curing temperature, optionally as defined by the temperature on the surface of the rotating drum, is from 120°C to 150°C.
7. The process according to any of the preceding embodiments, wherein the curing time in step (C) is in the range of 20 seconds to 10 minutes, preferably from 20 seconds to 60 seconds.
8. The process according to any of the preceding embodiments, wherein the moving belt is or comprises a composite material that is compatible with temperatures of up to 150°C, preferably up to 200°C and/or that has a static coefficient of friction of below 0.15, preferably below 0.1.
9. The process according to any of the preceding embodiments, wherein the moving belt is or comprises a polyfluorinated polymer, preferably polytetrafluorethylene, particularly preferred is or comprises fiberglass reenforced polytetrafluorethylene.
10. The process according to any of the preceding embodiments, wherein the moving belt rotates continuously in the overall system
11. The process according to any of the preceding embodiments, wherein heat bonding the absorbent pads onto the support layer in step (E) is achieved by heating the moving belt from below so that the temperature of the support layer is in the range of from 100°C to 180°C, preferably from 120°C to 160°C.
12. The process according to any of the preceding embodiments, wherein a pressure is applied onto the absorbent pad as the same is brought into contact with the backing layer in step (F).
13. The process according to any of the preceding embodiments, wherein the absorbent pad comprises a hydrophilic foam, in particular a hydrophilic polyurethane-based foam, or a hydrophilic non-woven material, in particular an airlaid material comprising polyvinylalcohol polymers (PVA), polyacrylic acid fibers or derivatized cellulose polymers, in particular carboxymethyl cellulose (CMC); or any combinations of these materials.
14. Wound dressing, in particular an island wound dressing, said dressing at least comprising:
   - at least one absorbent pad;
   - a vapor permeable backing layer overlaying said absorbent pad;
   - a perforated support layer underneath said absorbent pad, wherein said backing layer and said support layer extend beyond a periphery of said absorbent pad to define a surrounding border portion along said periphery of the absorbent pad;
      optionally wherein the backing layer and the support layer are at least partially heat bonded together in said border region, and wherein the absorbent pad and the support layer are at least partially heat bonded together;
   - a silicone gel layer on the side of the support layer that is opposite to the side that contacts the backing layer, wherein the silicone gel layer has openings that correspond to the perforations of the perforated support layer;
   - optionally a release layer covering the silicone gel layer and suitable to protect the same prior to use as a wound dressing.
15. The wound dressing according to embodiment 14, wherein the no adhesive, in particular no acrylic adhesive, is present between said backing layer and said support layer and wherein no adhesive, in particular no acrylic adhesive, is present between said absorbent pad and said support layer,
16. The wound dressing according to embodiment 14 or embodiment 15, wherein the silicone gel layer has been moulded onto the support layer, preferably has been obtained by applying a silicone mixture onto a perforated support layer and subsequently at least partially curing said silicone mixture to result in a at least partially cured, silicone gel, further preferably wherein the neither the silicone mixture nor the silicone gel has been perforated, in particular has not been exposed to any perforating elements.
17. The wound dressing according to any of embodiments 14 to 16, wherein the thickness of the silicone gel layer does not vary by more than 10%, preferably not by more than 7%, further preferably not by more than 5% across the entire lateral area of the silicone gel layer
18. The process or the wound dressing according to any of the preceding embodiments, wherein the support layer comprises a polyurethane polymer or is a layer of a polyurethane polymer.
19. The process or the wound dressing according to any of the preceding embodiments, wherein the support layer comprises a layer of a polyurethane polymer and a layer of a polyethylene polymer.
20. The process according to any of the preceding embodiments, wherein the template layer is made of thermoplastic polymer material that has a higher melting point, by at least 10 K, preferably by at least 20 K, than the polymer(s) making up the support layer.
21. The process according to embodiment 20, wherein the template layer comprises polypropylene (PP) polymer or is a layer of a polypropylene (PP) polymer.
22. The process or the wound dressing according to any of the preceding embodiments, wherein the backing layer and the perforated support layer are at least partially heat laminated together so that the adhesion strength between these two layers exceeds the peeling strength required to remove the entire wound dressing from a wound.
23. The process or the wound dressing according to any of the preceding embodiments, wherein the absorbent pad is a composite material comprising at least one foam material and at least one fiber material.

### Detailed Description of Embodiments of the Invention

In embodiments, the support layer is or comprises a thermoplastic polymer layer having a melting temperature of from 100°C to 150°C, preferably from 105°C to 135°C.

Preferably, the thermoplastic polymer is also elastomeric.

Preferred polymers for the support layer are polyurethane, polyethylene, ethylene vinyl acetate, polypropylene, polyvinyl chloride, polystyrol, polyether, polyester, polyamide, polycarbonate, polyether polyamide copolymers, polyacrylate, polymethacrylate, and/or polymaleate.

In preferred embodiments, the substrate layer comprises polyurethane, polyethylene or ethylene vinyl acetate. Polyurethane is particularly preferred.

In accordance with the present invention, any substrate layer or any combination of substrate sublayers may be used, as long as these layer(s) is/are suitable to be perforated . In particular, the substrate layer should be suitable to support a silicone mixture as applied in step (B) and the at least partially cured silicone gel resulting after step (C).

In embodiments, the thickness of said substrate layer is from 5 µm to 200 µm, preferably from 10 µm to 100 µm, further preferably from 15 µm to 60 µm.

In embodiments, the template layer is adapted such that it does not melt under the ultrasonic energy input as provided in the process of perforating the substrate layer. Thereby, deformed portions, or imprints, corresponding to the perforating elements may be provided without breaking the deformable film (e.g. by creating perforations therein or by complete loss of material).

In embodiments, the template layer is made of thermoplastic polymer material that has a higher melting point, by at least 10 K, preferably at least 20 K, than the polymer that is used as the supporting layer and that is perforated in said perforation step.

In embodiments, the melting point of the template layer is from 120 to 220°C, preferably from 130 to 190 °C

In embodiments, the template layer comprises or is a material selected from polypropylene (PP), polyester, polycarbonate or polyamide. Polypropylene is particularly preferred.

In embodiments, the thickness of the template layer is from 30 µm to 120 µm, preferably from 40 µm to 80 µm.

In embodiments, the backing layer preferably is or comprises polyurethane, in particular melt-blown polyurethane, which is optionally fed together with a further supporting layer, which supporting layer preferably comprises or consists of polyethylene.

Ultrasonic welding specifically for the purposes of creating perforations in a laminate (here: substrate) is known, in principle, for example from US 4 747 895, which discloses the ultrasonic perforating of a continuously moving strip of materials (for example an adhesive-backed plastic strip). In accordance with US 4 747 895, the continuously moving strip passes through a gap defined by an ultrasonic horn and is brought in contact with a rotating drum that has perforating projections acting as an array of perforation elements.

Silicone gels as used in the laminates and in the process of the present invention are understood to be gentle on the skin. This is because a soft silicone gel can follow the contours of the skin well thus providing a large contact surface area. Thus, although the actual adhesive force in each contact point of a silicone gel adhesive generally is less than that of a typical acrylic adhesive, the large surface area of contact achieved with a silicone gel affords a high overall adherence to the skin, whilst at the same time, the silicone gel is skin-friendly, i.e. when a silicone gel silicone gel is removed from the skin very few skin cells are co-removed due to the comparatively low adhesive force in each contact point. Therefore, the problem of skin stripping can be avoided or minimized.

In preferred embodiments, the silicone mixture resulting in the silicone gel after at least partially curing comprises or is a two-component addition- hardening silicone gel.

In case of this preferred silicone gel. the curing process (only) starts when the two components are joined with the substrate, in particular in step (C) as outlined above.

Such a two-component silicone gel may be a chemically crosslinked silicone gel, for example a polydimethyl siloxane gel, for instance a platinum catalyzed 2-component addition hardening RTV-silicone. Examples of gels that can be used are SilGel 612 from Wacker-Chemie GmbH, Burghausen, Germany, and MED-6340 from NuSil Technology, Carpinteria, USA. Examples of silicone gel gels useful in this context are also described in GB-A-2 192 142, GB-A-2 226 780 and EP-A1-0 300 620.

In embodiments of the present invention, in the final product, the "grammage" (amount of silicone gel per square meter) is from 10 g/m² to 500 g/m², preferably from 15 g/m² to 200 g/m².

In accordance with the present disclosure, the term *"heat bonding"* or *"heat laminating"* is understood as a process in which two layers comprising a polymer, in particular a thermoplastic polymer, are heated and softened to a point so that the two layers when brought into contact, in particular also under the application of outside pressure, for example as exerted by counterrotating rolls in a continuous process, form bonds, in particular a physical bonding, between each other that remain stable once the two softened or even partially melted polymeric materials are cooled again.

It is commonly understood that such a heat bonding or heat laminating leads to an adhesion strength between the layers heat bonded or laminated together so that the adhesion strength between the layers remains intact during the entirety of the dressing's intended use, in particular also when the dressing is removed from the wound (i.e. in a "wet" state).

In embodiments, the adhesion strength between the individual layers heat laminated together, even in a wet state, is greater than the peeling strength required to remove the entire dressing from the patient.

In aspects, the material of the absorbent pads is selected from a polymeric material capable of heat-laminating, i.e. forming permanent bonding when brought in contact with another polymer layer, in particular with a thermoplastic polymer layer.

In aspects, the absorbent pad is a hydrophilic foam, in particular a hydrophilic polyurethane-based foam, or a hydrophilic non-woven material, in particular an airlaid material based on polyvinylalcohol polymers (PVA), polyacrylic acid fibers or derivatized cellulose polymers, in particular carboxymethyl cellulose (CMC). Any combinations of the aforementioned materials are also included.

In aspects, the absorbent material comprises or consists of superabsorbent particles and/or superabsorbent fibers.

In aspects, the absorbent pad, in particular if said pad is or comprises gelling materials, is reinforced by non-gelling fibers or materials.

### List of reference signs:

- (1): support layer
- (1'): perforated support layer
- (2): silicone mixture layer (not cured)
- (2'): silicone gel (at least partially cured)
- (3): absorbent pad
- (4): backing layer
- (5): template layer
- (5'): deformed template layer
- (6): release liner (to be removed prior to use / for protective purpose only)
- (7): wound contact layer: perforated support layer + silicone gel layer (with aligned openings)
- (8): arrow showing the direction of dispensing of the silicone mixture
- (20), (20'): sacrificial processing aid layer
- (21): moving belt
- (21'): moving belt after delamination step
- (30): sonotrode of ultrasonic device
- (40): roll with perforation elements
- (45), (45'), (45"): heated transport rolls
- (46): heating zone
- (50): silicone mixture dispensing unit
- (60): rotating die cutter (RDC)
- (70): island dressing

## Claims

1. Process for the manufacture of wound dressings, said process at least comprising the steps of:
(A) feeding at least a template layer and a support layer through an ultrasonic perforation device,
which ultrasonic perforation device comprises a plurality of perforation elements, preferably an array of perforation elements, and which device is configured to introduce perforations, in particular an array of perforations, into at least said support layer;
wherein the perforating elements perforate the support layer, while, at the same time, deform the template layer without perforating the same;
(B) after completion of step (A), dispensing a silicone mixture onto the support layer so that the silicone mixture at least covers the non-perforated parts of the perforated support layer as present on top of the deformed template layer;
(C) at least partially curing the silicone mixture from step (B) to result in an at least partially cured silicone gel

2. The process of claim 1, wherein in step (C), the laminate from step (B) at least partially rotates on a heated rotating drum.

3. The process of claim 1 claim 2, further comprising at least one of the following steps, preferably at least further comprising steps (D) through (F):
(D) removing the laminate from step (C) from the heated rotating drum and/or separating the perforated support layer with the at least partially cured silicone gel of step (C) from the deformed template layer and, if present, from other optional layer(s), and transferring said separated laminate comprising the at least partially cured silicone gel layer on the perforated support layer onto a moving belt;
(E) continuously applying absorbent pads onto the laminate from step (D), said laminate comprising at least a perforated support layer and a cured silicone gel layer, wherein the absorbent pads are applied onto the side of the support layer that is not coated with silicone gel, wherein said absorbent pads are at least partially heat bonded to said side of the support layer not coated with silicone gel;
(F) continuously feeding a backing layer onto the laminate from step (E), wherein the backing layer is applied onto the absorbent pads and the side of the support layer that is not coated with silicone gel, wherein the backing layer is at least partially heat bonded to said side of the support layer that is not coated with silicone gel;
(G) cooling the laminate resulting from step (E) or from step (F) down to a temperature in a range of from 15°C to 40°C, preferably from 18°C to 25°C, wherein the cooled laminate is subsequently removed from the moving belt;
(H) continuously feeding and adding a release liner onto the silicone gel layer of the laminate as removed from the moving belt in step (G),
(I) at least partially heat bonding the backing layer onto the absorbent pad, optionally between two rotating rolls, wherein the upper roll, i.e. the roll in direct contact with the backing layer, is heated to a temperature in the range of from 120°C to 160°C.

4. The process according to any of the preceding claims, wherein the support layer is fed as a laminate, optionally as a laminate of polyurethane (PU) together with a reinforcing layer of polyethylene (PE).

5. The process according to any of the preceding claims, wherein the silicone mixture in dispensing step (B) is dispensed as an uncured or only partially cured two-component silicone gel mixture.

6. The process according to any of claims 2 to 5, wherein the curing time in step (C) is determined by adjusting one of the following, optionally by adjusting all of the following: temperature of the heated rotating drum, diameter of the heated rotating drum, speed of rotation of the rotating drum.

7. The process according to any of the preceding claims, wherein curing temperature, optionally as defined by the temperature on the surface of the rotating drum, is from 120°C to 150°C.

8. The process according to any of the preceding claims, wherein the curing time in step (C) is in the range of 20 seconds to 10 minutes, preferably from 20 seconds to 60 seconds.

9. Wound dressing, in particular an island wound dressing, said dressing at least comprising:
• at least one absorbent pad;
• a vapor permeable backing layer overlaying said absorbent pad;
• a perforated support layer underneath said absorbent pad, wherein said backing layer and said support layer extend beyond a periphery of said absorbent pad to define a surrounding border portion along said periphery of the absorbent pad;
• a silicone gel layer on the side of the support layer that is opposite to the side that contacts the backing layer, wherein the silicone gel layer has openings that correspond to the perforations of the perforated support layer;
wherein said silicone gel layer has been moulded onto said support layer.

10. The wound dressing according to claim 9, wherein the silicone gel layer has not been perforated, in particular has not been exposed to any perforating elements, in particular no mechanical perforation elements and no ultrasonic perforation elements.

11. The wound dressing according to claim 10, wherein the silicone gel layer has been obtained by applying a silicone mixture onto a perforated support layer and subsequently curing said silicone mixture to result in a at least partially cured, silicone gel.

12. The wound dressing according to any of claims 9 to 11, wherein the thickness of the silicone gel layer does not vary by more than 10%, preferably not by more than 7%, further preferably not by more than 5% across the entire lateral area of the silicone gel layer

13. The process or the wound dressing according to any of the preceding claims, wherein the support layer comprises a polyurethane polymer or is a layer of a polyurethane polymer.

14. The process or the wound dressing according to any of the preceding claims, wherein the support layer comprises a layer of a polyurethane polymer and a layer of a polyethylene polymer.

15. The process according to any one of claims 1 to 8, wherein the template layer is made of thermoplastic polymer material that has a higher melting point, by at least 10 K, preferably by at least 20 K, than the polymer(s) making up the support layer, optionally wherein the template layer comprises polypropylene (PP) polymer or is a layer of a polypropylene (PP) polymer.
